# DEMANDE DE BREVET EUROPEEN

(11) **EP 3 338 633 A1**
(43) Date de publication de la demande: **27.06.2018**
(21) Numéro de dépôt: 16306747.3
(22) Date de dépôt: 20.12.2016
(51) Int. Cl.: A61B 5/22, A61B 5/055

(54) **ERGOMETRE**

(71) Demandeur: UNIVERSITE DE BORDEAUX, 33000 Bordeaux (FR); Centre National de la Recherche Scientifique (CNRS), 75016 Paris (FR); Institut Polytechnique de Bordeaux, 33400 Talence (FR)
(72) Inventeur: SABATIER, Jocelyn, 33140 Villenave d'Ornon (FR); BOS, Frédéric, 33500 Libourne (FR); CAPORALE, Adrien, 33360 Latresne (FR); DELHOMME, Ludovic, 33500 Arveyres (FR); THÉVENOUX, Anne, 33000 Bordeaux (FR); MUNSCH, Fanny, 33200 Bordeaux (FR)
(74) Mandataire: Cabinet Plasseraud

(57) **Abrégé**

La présente invention concerne un ergomètre (10) comportant au moins un ensemble d'entraînement, chaque ensemble comprenant :
- un élément (11) d'entraînement,
- un vérin (12) hydraulique comportant une chambre de pression (32), ledit élément (11) d'entraînement étant relié audit vérin (12) hydraulique de sorte qu'une valeur de pression hydraulique appliquée dans ladite chambre de pression (32) détermine une force s'opposant au déplacement dudit élément (11) d'entraînement correspondant par un utilisateur, et
- une unité de régulation pour alimenter ladite chambre de pression (32) du vérin (12) hydraulique correspondant, en fluide hydraulique sous pression de sorte que ladite chambre de pression (32) présente une valeur de pression choisie pendant au moins une partie des exercices musculaires réalisés par l'utilisateur, ladite unité de régulation étant reliée à ladite chambre de pression (32) du vérin (12) hydraulique correspondant par un circuit d'alimentation (17, 18) en fluide hydraulique.

## Description

### ARRIERE-PLAN DE L'INVENTION

### Domaine de l'invention

La présente invention concerne le domaine des ergomètres.

Elle vise en particulier un ergomètre pour la réalisation d'exercices par une personne placée en position allongée dans un appareil de type imagerie par résonance magnétique (IRM) afin d'évaluer les propriétés mécaniques d'un ou plusieurs groupes de muscles de cet individu.

De façon non exhaustive, cette invention trouve également des applications dans différents autres domaines médicaux comme la cardiologie, la neurologie, l'angiologie ou encore l'orthopédie.

De manière plus générale, la présente invention trouve de nombreuses applications pertinentes dans le domaine médical, en recherche biomédicale et en recherche fondamentale.

### Arrière-plan technologique

Des tests peuvent être réalisés sur un individu afin d'apprécier ses fonctions musculaires, neurologiques, cardiaques, veineuses et artérielles, orthopédiques...

D'un point de vue biomédical, ces études peuvent être utiles pour investiguer les fonctionnements qui régissent la locomotion chez des sujets sains ou malades. Par exemple, chez un patient ayant subi un accident affectant les fonctions motrices au niveau central ou périphérique, un ergomètre pourrait permettre d'évaluer la perte ou la détérioration de ces fonctions. De même, l'utilisation d'un ergomètre pourrait permettre d'ajuster une prise en charge de la rééducation du patient.

Selon un autre exemple, un ergomètre permettrait d'étudier chez une personne alitée, ou chez une personne âgée, la perte musculaire susceptible de restreindre sa mobilité. L'idée étant de mieux connaître les facteurs induisant cette perte pour l'anticiper et mettre en place une prise en charge adéquate en vue de la minimiser, par exemple avec l'aide d'un kinésithérapeute.

Actuellement, les ergomètres de l'état de l'art offrent des utilisations limitées par rapport aux besoins.

Ainsi, de par leurs matériaux constitutifs, certains ergomètres ne peuvent être utilisés sur des équipements d'imagerie, pourtant incontournables dans le diagnostic de troubles moteurs.

En effet, il est connu que l'imagerie par résonnance magnétique (IRM) permet de visualiser les fonctions motrices au niveau musculaire, articulaire et en IRM fonctionnelle au niveau cérébral.

Or, un appareil IRM étant une machine émettant des champs magnétiques, les demanderesses ont constaté que l'association d'un ergomètre et d'un appareil d'imagerie par résonance magnétique est très délicate.

On constate que, même si un ergomètre de l'état de l'art comporte des pièces réalisées en matériau non ferromagnétique, les images acquises ne sont pas exemptes d'artéfacts. Notamment, on observe qu'au moins le système électrique de l'ergomètre est susceptible de perturber le fonctionnement de l'appareil IRM.

Or, un impératif est que l'ergomètre n'influe en rien sur la qualité des images acquises par l'appareil d'imagerie médicale.

Il faut donc que l'ergomètre soit construit à l'aide de pièces non ferromagnétiques validée comme ne créant pas de perturbations du champ magnétique, lesquelles entraineraient des dysfonctionnements à la fois de l'ergomètre et de l'appareil IRM.

La plupart des ergomètre présents sur le marché s'affranchisse de ces perturbations en déportant l'activité sur l'ergomètre dans une autre pièce.

Cependant, cette solution ne permet pas d'étudier par l'IRM, le corps humain en mouvement.

Les demanderesses ont également observé que les ergomètres existants sont souvent encombrants, difficilement déplaçables et manipulables par un seul opérateur qualifié.

Ainsi, l'ergomètre peut être placé à l'extrémité de l'appareil d'imagerie, en étant fixé solidairement à la table pour ne pas bouger lors de la réalisation d'exercices par un individu.

Or, un tel agencement de l'ergomètre est incompatible avec la nécessité d'une prise en charge rapide d'un patient placé dans l'appareil d'imagerie et ayant besoin de soins.

Par ailleurs, certains ergomètres ne peuvent être utilisés que pour un seul membre à la fois ou s'ils permettent de réaliser des exercices avec les deux membres, ils ne visent à appliquer qu'une même résistance sur chaque membre.

Tel est le cas, par exemple, d'un ergomètre comportant un dispositif de pédalier, dans lequel l'effort est imposé au patient par un disque de freinage sans régulation du couple de freinage.

D'autres sont difficilement utilisables en position allongée.

Enfin, aucun d'entre eux ne permet la synchronisation de la prise d'images avec l'exécution de l'exercice physique demandé.

Au surplus, ces ergomètres de l'état de l'art présentent des frottements statiques et cinétiques qui entrainent des pertes d'énergie. Celles-ci sont donc susceptibles de fausser les mesures.

Il existe donc un besoin pressant pour un ergomètre apte à l'examen d'un patient ou d'une personne dans un appareil d'imagerie à résonance magnétique, dont le principe de fonctionnement original surmonte les différents inconvénients mentionnés ci-dessus.

### Objet de l'invention

La présente invention vise un ergomètre, simple dans sa conception et dans son mode opératoire, ergonomique, polyvalent, paramétrable et pouvant se synchroniser avec le poste de commande afin de réaliser à l'aide d'imagerie, du diagnostic, du suivi thérapeutique, de la recherche biomédicale et des projets de recherche fondamentale.

L'objet également de la présente invention est qu'un tel ergomètre permette de mesurer avec précision les forces, les positions et les puissances exercées par un ou plusieurs groupes de muscles, tels que les muscles de flexion du pied, d'un individu.

Un autre objet de la présente invention est qu'un tel ergomètre soit apte à fonctionner dans un système d'imagerie par résonance magnétique (IRM), sans créer d'artefacts dans les données acquises.

Encore un objet de la présente invention est qu'un tel ergomètre soit d'une manipulation particulièrement aisée.

La présente invention concerne également un appareil de mesure pour la réalisation de diagnostics médicaux et/ou sportifs comprenant un système d'imagerie par résonance magnétique (IRM) et un tel ergomètre.

### BREVE DESCRIPTION DE L'INVENTION

A cet effet, l'invention concerne un ergomètre comportant au moins un ensemble d'entraînement, chaque ensemble comprenant :
- un élément d'entraînement,
- un vérin hydraulique comportant une chambre de pression, ledit élément d'entraînement étant relié audit vérin hydraulique de sorte qu'une valeur de pression hydraulique appliquée dans ladite chambre de pression détermine une force s'opposant au déplacement dudit élément d'entraînement correspondant par un utilisateur, et
- une unité de régulation pour alimenter ladite chambre de pression du vérin hydraulique correspondant, en fluide hydraulique sous pression de sorte que ladite chambre de pression présente une valeur de pression choisie pendant au moins une partie des exercices musculaires réalisés par l'utilisateur, ladite unité de régulation étant reliée à ladite chambre de pression du vérin hydraulique correspondant par un circuit d'alimentation en fluide hydraulique.

On entend par "fluide hydraulique", un fluide non visqueux défini par un coefficient de viscosité cinématique inférieure à 1,3.10⁻¹⁰ m²/s et un coefficient de compressibilité du fluide supérieur à 2.10⁹ N/m².

A titre purement illustratif, le fluide hydraulique est un produit aqueux, c'est-à-dire un liquide à base d'eau. De manière avantageuse, on ne mettra pas en oeuvre d'huiles minérales ou de synthèse.

L'utilisation de l'air induit, pour sa part, des difficultés de contrôle de régulation dues à la présence simultanée de frottements secs, de phénomènes de compressibilité et de vitesses élevées de circulation de l'air.

Pour chaque ensemble d'entraînement, la tige de piston du vérin hydraulique étant reliée à l'élément d'entraînement correspondant, la pression du fluide hydraulique présent dans la chambre de pression détermine l'effort résistant que devra vaincre l'utilisateur pour déplacer cet élément d'entraînement. L'unité de régulation alimentant cette chambre en fluide hydraulique sous pression via un circuit d'alimentation en fluide hydraulique, l'effort à réaliser est imposé à l'utilisateur uniquement par une distribution hydraulique.

On obtient ainsi un très bon contrôle sur la résistance imposée à l'individu lors de la réalisation d'exercices tout en ayant la possibilité de revenir aisément à une position standard de confort.

L'élément d'entraînement étant une pédale, celle-ci peut présenter un chausson pour recevoir le pied de l'utilisateur, ledit chausson étant fixé sur la pédale de manière à maintenir solidaire le pied avec la pédale.

Alternativement ou de façon complémentaire, on peut également prévoir un jeu de sangles pour le maintien du pied sur la pédale.

Avantageusement, l'ergomètre, selon la présente invention, comporte des moyens de réglage de chaque élément d'entraînement par rapport à un support portant le ou les éléments d'entraînement. Ces moyens de réglage peuvent, par exemple, permettre de régler en hauteur et/ou en profondeur la position de chaque élément d'entraînement pour ajuster l'ergomètre en fonction de la taille de l'utilisateur, et notamment la taille de la jambe de l'utilisateur et/ou de régler l'inclinaison du pied sur la surface d'appui de la plaque.

Différents modes de réalisation particuliers de cet ergomètre sont concevables, chacun ayant ses avantages particuliers et susceptibles de nombreuses combinaisons techniques possibles:
- Cet ergomètre comprend deux ensembles d'entraînement, chaque ensemble d'entraînement étant configuré pour mesurer au moins la force appliquée par chacun des membres de l'utilisateur.

A titre illustratif, cet utilisateur peut être un patient volontaire.

Bien entendu, l'ergomètre peut comporter un moyen pour bloquer l'élément d'entraînement d'un des deux ensembles, lorsque l'on cherche à réaliser des tests sur un seul membre de l'utilisateur.

A titre illustratif, cet ergomètre comprenant deux ensembles d'entraînement, chaque ensemble d'entraînement comprend un ensemble de capteurs destinés à mesurer la force appliquée par chacun des membres de l'utilisateur et la puissance de l'entraînement (puissance de pédalage).
- Cet ergomètre comportant plusieurs ensembles d'entraînement, lesdites unités de régulation sont distinctes de sorte que ledit ergomètre autorise l'application d'efforts dissymétriques à l'utilisateur.

Un tel mode de réalisation permet de moduler les efforts sur chaque membre d'un utilisateur. Notamment, un tel mode permet de moduler les efforts jusqu'à déterminer la limite à partir de laquelle le membre correspondant ne peut plus réaliser l'exercice. De préférence, l'ergomètre comportant deux ensembles d'entraînement, la valeur de pression choisie dans la chambre de pression du vérin hydraulique de chaque ensemble est différente. On applique ainsi une dissymétrie au niveau des éléments d'entraînement.
- Chaque élément d'entraînement est une pédale ou une poignée,
- Chaque sous-ensemble, ou groupe, comprenant un élément d'entraînement et ledit vérin hydraulique correspondant, est monté sur une plateforme, laquelle est montée sur un chariot mobile.

De préférence, cette plateforme est elle-même mobile entre une position déployée et au moins une position rétractée.

Ce chariot permet de simplifier la manutention et le déplacement du ou des sous-ensembles. Il est ainsi possible de venir positionner l'ergomètre aux deux extrémités d'une table type lit d'un système IRM ou de scanner ou de TEP scan.

De préférence, cette plateforme comporte un dispositif de maintien en position par aspiration pour bloquer en position celle-ci lorsque l'ergomètre est en fonctionnement.

A titre purement illustratif, ce dispositif de maintien en position peut comporter une pluralité de ventouses reliées à un système d'aspiration ou de vide.
- Chaque sous-ensemble, ou groupe, comprenant un élément d'entraînement et ledit vérin hydraulique correspondant, comporte également un capteur de force pour mesurer les forces et/ou un capteur de pression pour déterminer la pression du fluide dans ladite chambre de pression.

Pour mesurer la force exercée lors d'une flexion du pied de l'utilisateur, il est prévu avantageusement que le capteur de force soit du type piezorésistif ou à jauge. Avantageusement, l'ergomètre selon la présente invention comporte une unité informatique de traitement qui est apte à recevoir et traiter les signaux de mesure provenant du ou des capteurs afin de déterminer la force exercée par les muscles intervenant dans les mouvements exercés par l'utilisateur, ce dernier pouvant être un patient.
- Chaque circuit d'alimentation en fluide hydraulique est au moins en partie flexible pour autoriser un ajustement de la position de chaque sous-ensemble, ou groupe, comprenant un élément d'entraînement et ledit vérin hydraulique correspondant, par rapport à son unité de régulation,
- Chaque circuit d'alimentation en fluide comporte un système de déconnexion rapide permettant de séparer le sous-ensemble, ou groupe, comprenant un élément d'entraînement et ledit vérin hydraulique correspondant, de son unité de régulation.

Contrairement aux ergomètres du marché, un tel système de déconnexion rapide permet de séparer, en urgence, le sous-ensemble, ou groupe, comprenant un élément d'entraînement et ledit vérin hydraulique correspondant, de son unité de régulation.

Il est ainsi possible d'intervenir rapidement lorsqu'il est nécessaire d'extraire un patient de l'appareil d'imagerie médicale en cas de complications survenues pendant l'examen.
- Chaque unité de régulation comprend un vérin hydraulique comportant une chambre de pression, ladite chambre de pression étant en communication de fluide avec ledit circuit d'alimentation en fluide hydraulique et la tige du piston dudit vérin hydraulique étant reliée à un mécanisme d'entraînement configuré pour exercer une pression hydraulique prédéterminée dans la chambre de pression du vérin hydraulique de ladite unité de régulation.

De préférence, ce mécanisme d'entraînement comporte un moteur pas à pas possédant un arbre de moteur, la tige dudit piston étant solidaire d'une crémaillère, ou came crantée, reliée par une transmission à l'arbre dudit moteur pas à pas, lequel assure ainsi le déplacement de la crémaillère et la mise en pression du fluide dans ladite chambre de pression.

Le moteur pas à pas ayant un arbre de moteur, ce dernier est relié par une roue dentée sensiblement sans jeu à ladite crémaillère. Cette crémaillère est ainsi entraînée par le moteur pas à pas pour déterminer la valeur de pression du fluide hydraulique dans le vérin hydraulique.

Il comporte encore une unité de commande de moteur conçue pour fournir des signaux de fonctionnement au moteur pas à pas, laquelle est de préférence programmable selon au moins un paramètre tel que la résistance, le déplacement et/ou la puissance que doit offrir chaque élément d'entraînement lors de la réalisation de tests par l'utilisateur.

De préférence, cette unité de commande de moteur peut comporter au moins un programme d'ordinateur permettant de faire varier la force s'opposant au déplacement de l'élément d'entraînement par l'utilisateur de manière automatique, par exemple suivant une courbe de valeurs d'effort prédéfinie.

Cette unité de commande permet d'appliquer en fonction de la position angulaire de l'arbre de moteur, un déplacement de la crémaillère et par conséquent, une valeur de pression de fluide hydraulique par le déplacement linéaire de la tige de piston du vérin hydraulique correspondant.

De manière avantageuse, ce mécanisme d'entraînement comporte un dispositif de mesure du courant moteur pour déterminer le couple exercé par l'arbre de ce dernier pour maintenir en position ladite crémaillère.
- Un écran amagnétique entoure extérieurement au moins une partie de chaque unité de régulation, le reste de l'ergomètre étant réalisé en matériau amagnétique.

Un tel écran forme un blindage permettant avantageusement d'éviter de fausser les mesures réalisées par un dispositif d'imagerie tel qu'un système IRM.

Au moins certaines pièces peuvent être réalisées par impression tridimensionnelle par exemple en utilisant des matériaux plastiques tels que des thermoplastiques. A titre d'exemple, on pourra choisir le matériau parmi l'acrylonitrile butadiène styrène (ABS), le polycarbonate (PC), des polyamides, le polypropylène (PP). Ainsi, certaines des pièces des vérins hydrauliques telles que le piston, pourront avantageusement être réalisées par impression tridimensionnelle en polycarbonate (PC). Des procédés d'usinage et/ou de découpe et/ou de moulage peuvent être également utilisés pour fabriquer ces pièces.

Afin de renforcer des pièces d'usure, on pourra utiliser des inserts réalisés par exemple en aluminium ou à base d'aluminium, en laiton, en cuivre ou à base de cuivre.

De même, chaque circuit d'alimentation sera avantageusement réalisé en mettant en oeuvre un tuyau flexible en matière plastique ou en caoutchouc.

La présente invention concerne également un appareil de mesure pour la réalisation de diagnostics tels que médicaux et/ou sportifs, et pour la recherche.

Selon l'invention, cet appareil comprend un système d'imagerie par résonance magnétique (IRM) et un ergomètre tel que décrit précédemment.

Un tel appareil de mesure permet ainsi de faire réaliser des exercices à un individu en lui imposant un effort pendant une séance d'imagerie médicale de type IRM.

La présente invention concerne encore une méthode de mesure, selon laquelle on obtient en temps réel une pluralité d'informations caractérisant le fonctionnement physiologique ou physiopathologique d'un utilisateur réalisant des exercices sur un ergomètre tel que décrit précédemment. Cet utilisateur peut, bien entendu, être un patient ou un volontaire.

A titre purement illustratif, ces informations peuvent provenir d'une pluralité d'images obtenues par imagerie par résonance magnétique (IRM).

De manière avantageuse, cette méthode peut être utilisée pour étudier les zones motrices activées du cerveau ou l'appareil locomoteur d'un individu, lors de la réalisation d'exercices, en fonction de la résistance appliquée.

Elle trouve des applications également dans les domaines de la cardiologie, la neurologie, l'angiographie ou encore l'orthopédie.

Ainsi, l'objet de la présente invention, par ses différents aspects fonctionnels et structurels décrits ci-dessus, permet une mesure précise et fiable de la force produite par un ou des groupes de muscles, en évitant toute perturbation dans les mesures réalisées.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels :
- la Figure 1 montre de manière schématique un ergomètre pour réaliser des tests d'effort à un patient selon un premier mode de réalisation de la présente invention ;
- la Figure 2 est une vue élargie d'une unité de régulation de l'ergomètre de la Fig.1 ;
- la Figure 3 est une vue en coupe longitudinale de l'unité de régulation de la Fig. 2 et du vérin, la chambre de pression de l'unité de régulation étant reliée à la chambre de pression du vérin via un circuit d'alimentation en fluide hydraulique ;
- la Figure 4 est une vue schématique de l'ergomètre illustrant une partie hors du champ magnétique et une partie dans le champ magnétique ;
- la Figure 5 représente schématiquement l'ergomètre de la Fig. 1 disposé à l'avant d'un système IRM (Fig. 5a) et à l'arrière d'un système IRM (Fig. 5b).

### DESCRIPTION DETAILLEE DE MODE DE REALISATION DE L'INVENTION

Tout d'abord, on note que les figures ne sont pas à l'échelle.

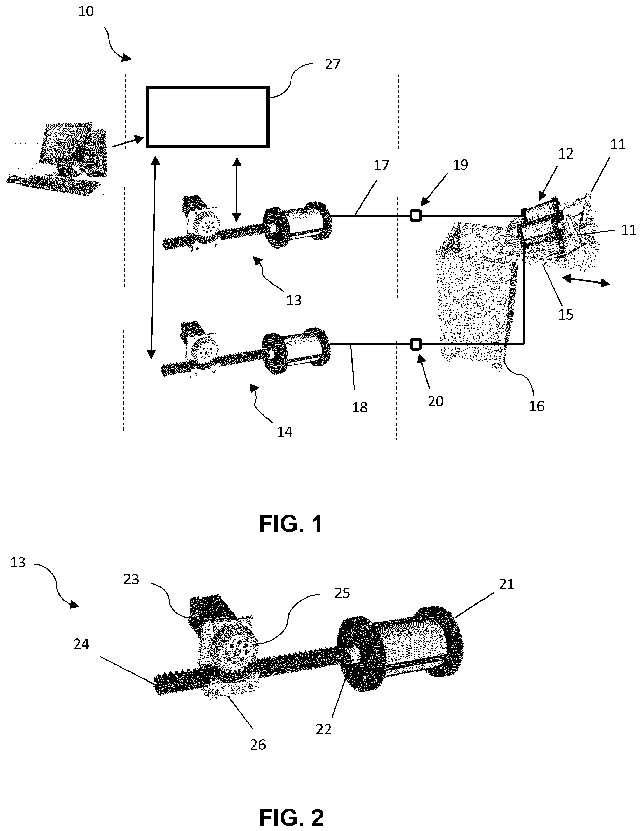

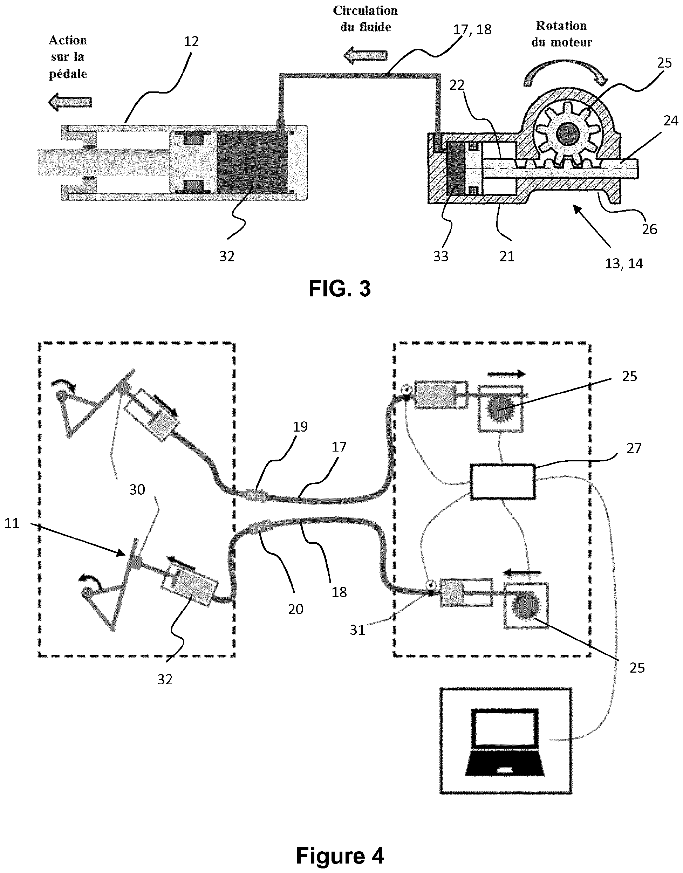

Un ergomètre selon un exemple de réalisation de la présente invention va maintenant être décrit dans ce qui va suivre en faisant référence conjointement aux figures 1 à 5.

Dans l'exemple décrit ici, l'ergomètre 10 comprend deux ensembles d'entraînement qui sont distincts et placés sensiblement en parallèle l'un de l'autre.

Chaque ensemble d'entraînement comporte une pédale 11 reliée à un vérin 12 hydraulique comportant une chambre de pression 32 de sorte qu'une valeur de pression hydraulique appliquée dans cette chambre de pression 32 détermine une force s'opposant au déplacement de la pédale 11 correspondante par le pied d'un utilisateur.

L'ergomètre comporte aussi un capteur de force 30 placé au niveau de chaque pédale 11 pour mesurer les forces appliquées. Cette information peut être utilisée pour réguler l'effort ou la puissance fournie par la pédale 11.

L'ergomètre peut comporter également un capteur de déplacement angulaire (non illustré) placé au niveau de chaque pédale 11 pour déduire la vitesse des pédales et donc la puissance de pédalage. Cette information peut être utilisée pour réguler la puissance mais également la position des pédales.

Chaque ensemble comporte également une unité 13, 14 de régulation pour alimenter cette chambre de pression 32 du vérin 12 hydraulique correspondant, en fluide hydraulique sous pression de sorte que ladite chambre de pression présente une valeur de pression choisie pendant au moins une partie des exercices réalisés par cet utilisateur.

Chaque sous-ensemble comprenant une pédale 11 et ses vérin 12 hydraulique et capteur de force 30 correspondants, est monté sur une même plateforme 15.

Cette plateforme 15 est elle-même supportée par un chariot 16 mobile pour assurer le déplacement de ce dernier. Ce chariot 16, qui est typiquement placé au niveau de l'extrémité de la table du système d'imagerie par résonance magnétique (IRM), peut ainsi être manipulé facilement par un opérateur.

La plateforme 15 est montée mobile sur le chariot 16 via des glissières 34, 35 entre une position déployée dans laquelle la plateforme 15 est placée sur la table de d'IRM pour positionner les pédaliers sur cette table, et une position rétractée dans laquelle la plateforme 15 est retirée de l'extrémité de cette table.

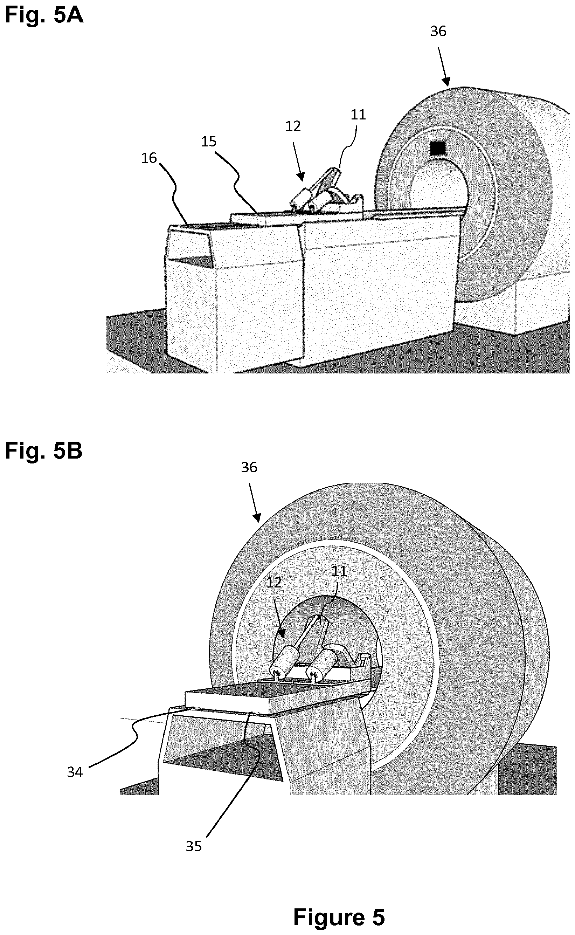

Comme l'illustrent les figures 5A et 5B, le chariot supportant le pédalier peut être placé à l'avant de l'IRM 36 (figure 5A) ou à l'arrière de l'IRM 36 (figure 5B).

Cette plateforme 15 comporte également une pluralité de ventouses reliées à un dispositif d'aspiration (non représentés) pour assurer un blocage en position aisé de cette plateforme sur la table de l'IRM 36 lorsque l'ergomètre 10 est utilisé.

Chaque unité 13, 14 de régulation est reliée à la chambre de pression 32 du vérin 12 hydraulique correspondant par un circuit 17, 18 souple d'alimentation en fluide hydraulique.

Ces circuits 17, 18 d'alimentation autorisent ainsi un ajustement de la position du chariot 16 par rapport aux unités 13, 14 de régulation qui sont placées à distance du tunnel du système d'imagerie par résonance magnétique. A titre purement illustratif, ces unités 13,14 de régulation peuvent être placées à plusieurs mètres de l'entrée du mini-tunnel.

Dans le cas de l'IRM, le champ magnétique généré décroit en fonction de la distance. On s'assure ainsi avantageusement de l'absence de perturbation induite par ce champ magnétique sur les unités 13, 14 de régulation.

Chaque circuit 17, 18 d'alimentation en fluide hydraulique comporte un système 19, 20 de déconnexion rapide permettant de séparer chaque sous-ensemble de son unité 13, 14 de régulation correspondante. A titre d'exemple, un tel système 19, 20 de déconnexion rapide comporte un raccord à déconnexion rapide étanche.

Chaque unité 13, 14 de régulation comprend un vérin 21 hydraulique comportant une chambre de pression 33, cette chambre de pression étant en communication de fluide avec son circuit 17, 18 d'alimentation en fluide hydraulique.

La tige 22 du piston de chaque vérin 21 hydraulique est reliée à un mécanisme d'entraînement configuré pour exercer une pression hydraulique prédéterminée dans la chambre de pression du vérin 21 hydraulique de ladite unité 13, 14 de régulation correspondante.

Ce mécanisme d'entraînement comporte ici un moteur 23 pas à pas possédant un arbre de moteur, la tige 22 du piston du vérin hydraulique correspondant étant solidaire d'une came 24 crantée.

Cette came 24 crantée est elle-même reliée par une roue 25 dentée à l'arbre du moteur 23 pas à pas, lequel assure ainsi le déplacement de la came 24 crantée et la mise en pression du fluide dans la chambre de pression 33 du vérin hydraulique correspondant.

Un guide 26 permet d'assurer un déplacement linéaire de la came 24 crantée.

Une électronique 27 permet de commander chaque moteur 23 pas à pas, une unité de programmation (non représentée) permettant par exemple de varier la résistance appliquée au niveau de chaque pédale 11 par l'application d'une pression hydraulique déterminée dans le circuit 17, 18 d'alimentation hydraulique s'étendant entre les chambres de pression des deux vérins 12, 21 hydrauliques de chaque système d'entraînement.

Afin d'éviter toute perturbation dans les mesures réalisées par un système d'imagerie par résonance magnétique, un écran amagnétique (non représentée) entoure extérieurement chaque unité 13, 14 de régulation, le reste de l'ergomètre étant réalisée en matériau amagnétique.

L'ergomètre comprend un ensemble de capteurs 30, 31 disposés au niveau des pédales, des vérins et des moteurs.

Selon une forme de réalisation, une paire de capteurs de pression de fluide est disposée au niveau des chambres, une paire de capteur de position et de vitesse au niveau des pédales et une paire de capteurs de courant au niveau des moteurs.

Selon un mode opératoire, les informations des capteurs 30, 31 sont transmises à l'électronique 27 qui les retransmet à un poste de contrôle muni d'un écran d'affichage.

Suite aux informations affichées sur le poste de contrôle, un opérateur peut envoyer une consigne pour contrôler la force renvoyée par les moteurs aux pédales via les unités de régulation 13, 14, afin de réguler la position, ou la vitesse ou la puissance au niveau de la pédale.

## Revendications

1. Ergomètre (10) comportant au moins un ensemble d'entraînement, chaque ensemble d'entraînement comprenant :
- un élément (11) d'entraînement,
- un vérin (12) hydraulique comportant une chambre de pression (32), ledit élément (11) d'entraînement étant relié audit vérin (12) hydraulique de sorte qu'une valeur de pression hydraulique appliquée dans ladite chambre de pression (32) détermine une force s'opposant au déplacement dudit élément (11) d'entraînement correspondant par un utilisateur, et
- une unité (13, 14) de régulation pour alimenter ladite chambre de pression (32) du vérin (12) hydraulique correspondant, en fluide hydraulique sous pression de sorte que ladite chambre de pression (32) présente une valeur de pression choisie pendant au moins une partie des exercices musculaires réalisés par l'utilisateur, ladite unité (13, 14) de régulation étant reliée à ladite chambre de pression (32) du vérin (12) hydraulique correspondant par un circuit (17, 18) d'alimentation en fluide hydraulique.

2. Ergomètre (10) selon la revendication 1, **caractérisé en ce qu'**il comprend deux ensembles d'entraînement, chaque ensemble d'entraînement comprenant un ensemble de capteurs destinés à mesurer la force appliquée par chacun des membres de l'utilisateur et la puissance de l'entraînement.

3. Ergomètre (10) selon la revendication 1 ou 2, **caractérisé en ce que** ledit ergomètre comportant plusieurs ensembles d'entraînement, lesdites unités (13, 14) sont distinctes de sorte que ledit ergomètre autorise l'application d'efforts dissymétriques à l'utilisateur.

4. Ergomètre (10) selon l'une quelconque des revendications 1 à 3, **caractérisé en ce que** chaque élément d'entraînement est une pédale ou une poignée.

5. Ergomètre (10) selon l'une quelconque des revendications 1 à 4, **caractérisé en ce que** chaque sous-ensemble comprenant un élément (11) d'entraînement et ledit vérin (12) hydraulique correspondant, est monté sur une plateforme (15), de préférence mobile entre une position déployée et au moins une position rétractée, laquelle est montée sur un chariot (16) mobile.

6. Ergomètre (10) selon la revendication 5, **caractérisé en ce que** ladite plateforme (15) comporte un dispositif de maintien en position par aspiration pour bloquer en position celle-ci lorsque l'ergomètre est utilisé.

7. Ergomètre (10) selon l'une quelconque des revendications 1 à 6, **caractérisé en ce que** chaque sous-ensemble comprenant un élément (11) d'entraînement et ledit vérin (12) hydraulique correspondant, comporte également un capteur de force pour mesurer les forces et/ou un capteur de pression pour déterminer la pression du fluide dans ladite chambre de pression (32).

8. Ergomètre (10) selon l'une quelconque des revendications 1 à 7, **caractérisé en ce que** chaque circuit (17, 18) d'alimentation en fluide hydraulique est au moins en partie flexible pour autoriser un ajustement de la position de chaque sous-ensemble comprenant un élément (11) d'entraînement et ledit vérin (12) hydraulique correspondant, par rapport à son unité (13, 14) de régulation.

9. Ergomètre (10) selon l'une quelconque des revendications 1 à 8, **caractérisé en ce que** chaque circuit (17, 18) d'alimentation en fluide comporte un système de déconnexion rapide (19, 20) permettant de séparer le sous-ensemble comprenant un élément (11) d'entraînement et ledit vérin (12) hydraulique correspondant, de son unité (13, 14) de régulation.

10. Ergomètre (10) selon l'une quelconque des revendications 1 à 9, **caractérisé en ce que** chaque (13, 14) unité de régulation comprend un vérin (21) hydraulique comportant une chambre de pression (33), ladite chambre de pression (33) étant en communication de fluide avec ledit circuit d'alimentation en fluide hydraulique (17, 18) et la tige (22) du piston dudit vérin (21) hydraulique étant reliée à un mécanisme d'entraînement configuré pour exercer une pression hydraulique prédéterminée dans la chambre de pression (33) du vérin (21) hydraulique de ladite unité de régulation correspondante.

11. Ergomètre (10) selon la revendication 10, **caractérisé en ce que** ledit mécanisme d'entraînement comporte un moteur (23) pas à pas possédant un arbre de moteur, la tige dudit piston étant solidaire d'une crémaillère (24) reliée par une transmission à l'arbre dudit moteur pas à pas, lequel assure ainsi le déplacement de la crémaillère (24) et la mise en pression du fluide dans ladite chambre de pression (33).

12. Ergomètre (10) selon la revendication 11, **caractérisé en ce qu'**il comporte un dispositif de mesure du courant moteur pour déterminer le couple exercé par l'arbre de ce dernier pour maintenir en position ladite crémaillère.

13. Ergomètre (10) selon l'une quelconque des revendications 1 à 11, **caractérisé en ce qu'**un écran amagnétique entoure extérieurement au moins une partie de chaque unité de régulation, le reste de l'ergomètre étant réalisé en matériau amagnétique.

14. Appareil de mesure pour la réalisation de diagnostics et pour la recherche, **caractérisé en ce qu'**il comprend un système d'imagerie par résonance magnétique (IRM) et un ergomètre selon l'une quelconque des revendications 1 à 13.

15. Méthode de mesure, **caractérisée en ce qu'**on obtient en temps réel une pluralité d'informations caractérisant le fonctionnement physiologique ou physiopathologique d'un utilisateur réalisant des exercices sur un ergomètre selon l'une quelconque des revendications 1 à 13.
